Europäisches Patentamt

**European Patent Office**

**Office européen des brevets**

(19)

(11) Publication number: **0 063 657**
A1

## EUROPEAN PATENT APPLICATION

(12)

(21) Application number: **81301893.4**

(22) Date of filing: **29.04.81**

(51) Int. Cl.³: **A 61 K 9/06,** A 61 K 9/70,
A 61 K 9/02, A 61 K 31/70

(43) Date of publication of application: **03.11.82**
**Bulletin 82/44**

(71) Applicant: **MERCK & CO. INC., 126, East Lincoln Avenue
P.O. Box 2000, Rahway, New Jersey 07065 (US)**

(72) Inventor: **Field, Arthur Kirk, 376 Meadowbrook Road,
North Wales M.R. 1 Pennsylvania 19454 (US)**
Inventor: **Harwood, Richard J., 3219 Adams Court North,
Bensalem Pennsylvania 19020 (US)**

(84) Designated Contracting States: **AT BE CH DE FR GB IT
LI LU NL SE**

(74) Representative: **Crampton, Keith John Allen et al, D
YOUNG & CO 10 Staple Inn, London WC1V 7RD (GB)**

(54) **Topical application of interferon inducers.**

(57) Virus infections of eye, skin and mucous membrane
tissue are effectively treated by controlled-release topical
application of interferon inducers. The inducers are
included in pharmaceutical compositions with a controlled-
release agent such as a pharmaceutically acceptable water-
soluble polymer that is solid at body temperature, e.g. a
cellulose ether.

EP 0 063 657 A1

- 1 -                                    16292

## TOPICAL APPLICATION OF INTERFERON INDUCERS

This invention is concerned with the prevention and treatment of virus infections of eye, skin and mucous membrane tissue by the controlled release topical application of interferon inducers.

Interferon is a class of soluble proteins that inhibit virus multiplication. They are produced by cells in response to a virus infection and are not virus-specific in their inhibitory actions.

The cellular production of interferon also can be induced by certain materials other than viruses such as double-stranded synthetic polynucleotide complexes (dsRNA) as reported by Field et al., Proc. Nat. Ac. Sci., 58, 1004-1010 (1967), and U.S. Patent 4,124,702, especially polyriboinosinic acid:polyribocytidylic acid. This material is otherwise known as poly I:poly C, poly I:C, poly rI:poly rC, or $rI_n \cdot rC_n$ and is hereinafter referred to as poly I:C.

Furthermore, dsRNA such as poly I:C have been successfully used to induce resistance against systemic as well as localized infections. Topical application of drugs, including interferon inducers, often requires frequent repeated treatments in order to ensure adequate drug exposure of the infected tissue.  This limitation has been a serious problem, especially for potential application of antiviral drugs to mucous membranes and the cornea and conjunctiva of the eye.

The present invention is based on the unexpected discovery that controlled release topical application of interferon inducers is very effective in the local topical treatment of virus infections of the eye, skin and mucous membrane tissue, and in accordance with the present invention an interferon inducer is incorporated in a controlled-release means for effecting such treatment.

The present invention also provides a controlled release formulation for topical application to the skin, eye or mucous membrane tissue comprising a pharmaceutically acceptable water-soluble polymer that is solid at body temperature and in which is incorporated, in a homogeneous manner, a therapeutically effective antiviral amount of an interferon inducer.  Treating virus infections of the eye, skin and mucous membrane tissue by topical application of an interferon inducer using a controlled release means can provide an optimum amount of interferon inducer at the site of the infection for a prolonged period of time.

Interferon inducers useful in the novel method of treatment of this invention are double-stranded synthetic polynucleotide complexes such as poly I:C and poly A:U;  natural double-stranded RNA derived from virus-infected bacterial cells, such as MU9-dsRNA or dsRNA isolated from Penicillium species; and non-polynucleotide inducers such as Tilorone hydrochloride and 1,3-dimethyl-4-(3-dimethylamino-propylamino)-1H-pyrazolo-[3,4-b]-quinoline dihydrochloride.

The controlled release of an interferon inducer is accomplished by incorporating the interferon inducer, in a homogeneous manner, into a matrix consisting of a water-soluble polymer that is solid at or near body temperature. The water-soluble polymer may be a cellulose ether such as methylcellulose, hydroxyethylcellulose, hydroxypropylcellulose, hydroxypropylmethylcellulose, or sodium carboxymethylcellulose, or other pharmaceutically acceptable polymer such as polyvinyl alcohol, polyvinylpyrrolidone, carboxyvinylpolymer or polyethylene glycol. The preferred water-soluble polymer is hydroxypropylcellulose. Amounts of 0.0025 to 0.4 mg of interferon inducer per 1 mg of formulation are appropriate, preferably 0.075 to 0.25 mg of interferon inducer per 1 mg of formulation.

For treatment of virus infections of the skin, such as by the Herpes group of viruses, it is convenient to employ the novel formulation of this invention in the form of a sheet with a thickness of about 0.25 to about 3.0 mm, preferably about 1 mm with an area approximating the area to be treated and held in place by bandaging materials.

An alternate means of accomplishing the novel controlled release application of this invention on the skin is by use of a specially designed bandage for the purpose, such as disclosed in U.S. Patents 3,734,097 and 3,797,494, and U.S. Application Serial No. 960,354, filed November 13, 1978 by Harwood.

For treatment of virus infections of the eye, rectangular or rod shaped pieces of the formulation of about 2.5-12 mg may be inserted between the eye and the lower eyelid.

For use as vaginal inserts or rectal suppositories, the controlled release formulations of this invention are in the usual sizes and shapes and contain between about 2.5-250 mg of interferon inducer per 1 g of formulation.

The following Examples describe two experiments comparing the beneficial effect of the novel method of treatment of this invention in the eye, with certain control treatments, and a typical ophthalmic insert formulation.

From the results it is concluded that rabbit eye treatment using a poly I:C insert was effective in preventing or substantially delaying and reducing the Herpes virus infection, as measured by reduction of virus production. This protective effect was the result of effective controlled delivery of the interferon inducer, poly I:C. Treatment of animals with either placebo inserts or poly I:C in PBS did not effectively alter virus infection.

## EXAMPLE 1

New Zealand white rabbits were infected with Herpes simplex virus Type 1 (HSV-1) by dropping 0.05 ml containing approximately 10 infectious doses onto the cornea of each eye. Animals were treated (2 animals per group, four eyes total) with:

    (1)   200 µg of poly I:C in 0.1 ml of phosphate buffered saline (PBS);

    (2)   0.1 ml of phosphate buffered saline; or

    (3)   eye inserts each containing 400 µg of poly I:C.

The first treatment occurred 6 hours prior to infection and continued once daily for 4 days. Prior to treatment each day, each eye was swabbed to recover infectious virus, and the HSV-1 titer was determined in each case and the results are recorded in Table I.

TABLE I

Rabbit Eye Insert - Experiment 1

| Rabbit Eye Treatment | Animal Treated | | Herpes Simples Virus Titer on Day [a] | | | | |
|---|---|---|---|---|---|---|---|
| | ID Number | Eye | 0 (Pre) | 1 | 2 | 3 | 4 |
| Placebo (PBS) | 3 | L | -0.3 | 2.0 | 3.4 | 3.4 | 2.7 |
| | | R | -0.3 | 2.0 | 3.9 | 2.9 | 3.4 |
| | 4 | L | -0.3 | 0.7 | 4.0 | 3.0 | 2.9 |
| | | R | -0.3 | -0.3 | 3.0 | 2.4 | 3.4 |
| | GMT | | -0.3 | 1.1 | 3.6 | 2.9 | 3.1 |
| Poly I:C in PBS | 1 | L | -0.3 | -0.3 | 2.0 | 1.9 | 1.4 |
| | | R | -0.3 | 1.0 | 3.4 | 3.0 | 2.9 |
| | 2 | L | -0.3 | 1.9 | 2.4 | 2.7 | 3.4 |
| | | R | -0.3 | -0.3 | 1.7 | 3.0 | 3.4 |
| | GMT | | -0.3 | 0.6 | 2.4 | 2.7 | 2.8 |
| Poly I:C Insert | 5 | L | -0.3 | -0.3 | -0.3 | -0.3 | -0.3 |
| | | R | -0.3 | -0.3 | -0.3 | -0.3 | -0.3 |
| | 6 | L | -0.3 | -0.1 | 3.7 | 4.4 | 3.0 |
| | | R | -0.3 | -0.3 | -0.3 | -0.3 | -0.3 |
| | GMT | | -0.3 | -0.3 | 0.7 | 0.9 | 0.5 |

GMT = log Geometric Mean Titer

[a] Titers are expressed as $\log_{10}$ of the infectious virus
concentration ($TCID_{50}$) determined following exposure of
cultures of guinea pig embryo cells to serial 10-fold
dilutions of virus samples and incubation at 36°C for
5 days.  -0.3 was assigned to those samples containing
no infectious virus.

16292

- 8 -                                    16292

## EXAMPLE 2

A second experiment was conducted substantially as described in Example 1, but employing the following treatment groups of 2 rabbits each:

(1) eye inserts each containing approximately 400 µg of poly I:C;

(2) placebo eye inserts;

(3) 0.1 ml of phosphate buffered saline

The HSV-1 titers are shown in Table II.

TABLE II

Rabbit Eye Insert - Experiment 2

| Rabbit Eye Treatment | Animal Treated | | Herpes Simplex Virus Titer on Day[a] | | | |
|---|---|---|---|---|---|---|
| | ID Number | Eye | 1 | 2 | 3 | 4 |
| Placebo (PBS) | 5 | L | 0.0 | 1.7 | 3.4 | 3.7 |
| | | R | -0.3 | -0.3 | 1.9 | 3.9 |
| | 6 | L | -0.3 | -0.3 | 0.4 | 2.7 |
| | | R | -0.3 | 0.0 | 2.7 | 3.0 |
| | GMT | | -0.2 | 0.3 | 2.1 | 3.3 |
| Insert Placebo | 3 | L | -0.3 | 2.0 | 4.0 | 3.9 |
| | | R | -0.3 | 1.4 | 3.9 | 4.0 |
| | 4 | L | -0.3 | 2.7 | 3.0 | 3.7 |
| | | R | 0.0 | 1.0 | 0.9 | 3.0 |
| | GMT | | -0.2 | 1.8 | 3.0 | 3.7 |
| Poly I:C Insert | 1 | L | -0.3 | 0.9 | 0.9 | 1.9 |
| | | R | -0.3 | 0.7 | -0.3 | -0.3 |
| | 2 | L | 0.4 | 0.0 | 0.7 | -0.3 |
| | | R | -0.3 | 0.7 | 1.0 | 1.7 |
| | GMT | | -0.1 | 0.6 | 0.6 | 0.8 |

16292

0063657

GMT = log Geometric Mean Titer

[a] Titers are expressed as $\log_{10}$ of the infectious virus concentration ($TCID_{50}$) determined following exposure of cultures of guinea pig embryo cells to serial 10-fold dilutions of virus samples and incubation at 36°C for 5 days. -0.3 was assigned to those samples containing no infectious virus.

## EXAMPLE 3

Controlled Release Formulation of Poly I:C Adapted
For Use As An Eye-Insert

| | |
|---|---|
| Polyribocytidylic acid potassium salt | 225 mg |
| Polyriboinosinic acid potassium salt | 216 mg |
| Hydroxypropylcellulose (Klucel HF) | 3970 mg |

1. Add polyribocytidylic acid potassium salt to 10 ml of distilled water. Result is a thick gel. "Solution A".

2. Add polyriboinosinic acid potassium salt to 10 ml of distilled water. Result is a thick gel. "Solution B".

3. Store Solution A and Solution B at 5°C for 16 to 18 hours.

4. Place 100 g of distilled boiling water in 250 ml beaker.

5. Slowly add all of the hydroxypropylcellulose to the hot water.

6. Stir the slurry of hydroxypropylcellulose until a gel forms. Allow the gel to cool to room temperature. "Gel C". Store Gel C at 5°C for 16 to 18 hours.

7. Combine Solution A, Solution B and Gel C in a small desicating dish. "Gel D".

8.  Freeze dry Gel D to a dry cake.

9.  Compress the dry cake into a flat sheet which is 1 mm thick.

10. Cut pieces from the 1 mm film which are rectangular in shape and which weigh approximately 10 mg each (actual weight approximately 11.5 mg).

11. Store pieces at 5°C until used.

13

## CLAIMS

1.      A controlled release formulation for topical application to the skin, eye or mucous membrane tissue comprising a pharmaceutically acceptable water-soluble polymer that is solid at body temperature and in which is incorporated, in a homogeneous manner, a therapeutically effective antiviral amount of an interferon inducer.

2.      A formulation as claimed in Claim 1, in which the water-soluble polymer is a cellulose ether.

3.      A formulation as claimed in Claim 2, in which the cellulose ether is hydroxypropylcellulose.

4.      A formulation as claimed in Claim 1, 2 or 3, in which the interferon inducer is a synthetic double-stranded RNA, a natural double-stranded RNA, or a non-polynucleotide interferon inducer.

5.      A formulation as claimed in Claim 4, in which the interferon inducer is poly I:C.

6.      A formulation as claimed in Claim 5 in a form suitable for use on the eye.

7.      An interferon inducer in a controlled release means for use in the infections of skin, eye and mucous membrane tissue by topical application.

8.      A synthetic double-stranded RNA, a natural double-stranded RNA, or a non-polynucleotide interferon inducer in a controlled release means for use in the infections of skin, eye and mucous membrane tissue by topical application.

9.      Poly I:C in a controlled release means for use in the infections of skin, eye and mucous membrane tissue by topical application.

## DOCUMENTS CONSIDERED TO BE RELEVANT

| Category | Citation of document with indication, where appropriate, of relevant passages | Relevant to claim |
|---|---|---|
| X | BIOLOGICAL ABSTRACTS, vol. 65, no. 12, 1978, abstract 69208, PHILADELPHIA (US) H.B. LEVY et al.: "Topical treatment of vaccinia virus infection with an interferon inducer in rabbits" & J. Infect Dis 137(1): 78-80,1978<br><br>* the whole abstract *<br>--- | 1-9 |
| X | US - A - 4 039 656 (BAYER)<br><br>* column 2, lines 32-40; column 3, lines 30-35, line 67 to column 4, line 12; column 5, lines 22-31; claims 1 and 2 *<br>--- | 1-9 |
| | FR - A - 2 271 824 (THE UPJOHN COMPANY)<br><br>* page 1, line 24 to page 2, line 10; page 3, lines 6-15; page 6, line 31 to page 7, line 3; example 6, page 16 *<br>& GB - A - 1 475 221<br>--- | 1-9 |
| | DE - A - 2 051 745 (UGINE KUHLMANN)<br><br>* example; pages 30-31; claims 1-7 *<br>& GB - A - 1 294 580<br>--- | 1-9 |
| | FR - A - 2 073 366 (GLAXO LAB.)<br><br>* page 12, line 33 to page 13, line 10; claims 1, 38 and 39 *<br>& GB - A - 1 331 933<br>--- | 1-9 |

./..

X The present search report has been drawn up for all claims

| Place of search | Date of completion of the search | Examiner |
|---|---|---|
| The Hague | 08.01.1982 | GERMINARIO |

EPO Form 1503.1  06.78

**CLASSIFICATION OF THE APPLICATION (Int. Cl. 3)**

A 61 K   9/06
         9/70
         9/02
        31/70

**TECHNICAL FIELDS SEARCHED (Int.Cl. 3)**

A 61 K  31/70
         9/06
         9/02
         9/70

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

European Patent
Office

**EUROPEAN SEARCH REPORT**

0063657

Application number

EP 81 30 1893

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | FR - A - 2 162 637 (BAYER A.G.)<br><br>* page 3, line 7 to page 4, line 34; claims 1-3 *<br>& GB - A - 1 356 076 | 1-9 | |
| | FR - M - 8373 (MERCK & CO.)<br><br>* page 1, line 14 to page 2, line 27; page 47, line 5 to page 49, line 21 * | 1-9 | |
| | FR - A - 2 158 488 (BEECHAM GROUP)<br><br>* page 3, lines 2-34; page 9, lines 4-9; claims 1,19,20 *<br>& GB - A - 1 410 013 | 1-9 | TECHNICAL FIELDS SEARCHED (Int. Cl.³) |
| | FR - A - 2 276 811 (SCHERING)<br><br>* claims 1-15 *<br>& GB - A - 1 510 999 | 1-9 | |
| | FR - A - 1 296 518 (J.F. CHAUVIN)<br><br>* the whole document * | 1-9 | |
| A | FR - A - 2 197 582 (D.L. MAYER)<br><br>* page 2, lines 19-31; page 3, lines 22,23; page 4, lines 1-10 *<br>& US - A - 3 855 772 | 1-9 | |

EPO Form 1503.2   06.78